# EUROPEAN PATENT APPLICATION

(11) **EP 1 388 337 A2**
(43) Date of publication of application: **11.02.2004**
(21) Application number: 03254578.2
(22) Date of filing: 22.07.2003
(51) Int. Cl.: A61J 7/04, G06F 19/00

(54) **Pharmaceutical dispenser system**

(30) Priority: 05.08.2002 US 213146
(71) Applicant: Hewlett-Packard Development Company, L.P., Houston, TX 77070 (US)
(72) Inventor: Greeven, John C., Corvallis, OR 97330 (US); Valley, Jeffrey M., Corvallis, OR 97330 (US)
(74) Representative: Carpmaels & Ransford

(57) **Abstract**

A pharmaceutical dispenser system (10) comprising a container (30, 130) configured to store discrete doses of pharmaceuticals and instructional indicia (34,134) configured to align with each discrete dose of pharmaceutical indicating a time such discrete dose is to be administrated in accordance with a patient-specific therapy regimen.

## Description

### BACKGROUND

A significant number of prescriptions are not properly taken annually. For example, patients on a prescription therapy may take an improper dose, or may forget to take a dose of the medication at the proper time. Other patients may not remember when they last took their medication or when they are to take the next dose. In some therapies, this can present a significant problem, particularly where doses of the medication vary over the course of the treatment regimen. If a patient misses doses, or takes the wrong dose at the wrong time during the treatment regimen, adverse side-effects, as well as reduced efficacy, may result.

Containers thus have been developed to store doses of medication to aid patients in taking their medication on time. For example, containers with seven spaces labeled for each day of the week are known. Some containers further define AM and PM spaces labeled for each day of the week to remind patients when to take their medications. One drawback to these systems, however, is that the patient, or a caregiver, must manually fill the containers with the correct medications before the containers can be used.

### SUMMARY

A pharmaceutical dispenser system comprising a container configured to store discrete doses of pharmaceuticals and instructional indicia configured to align with each discrete dose of pharmaceutical indicating a time such discrete dose is to be administrated in accordance with a patient-specific therapy regimen.

### BRIEF DESCRIPTION OF THE DRAWINGS

Fig. 1 is a somewhat schematic representation of a system configured to produce a container with discrete dosing instructions recorded thereon.
Fig. 2 is a pharmaceutical container produced by the system of Fig. 1, the container having discrete doses with associated discrete dosing instructions.
Fig. 3 is an isometric view of an alternative embodiment pharmaceutical container, again having discrete doses with associated discrete dosing instructions.
Fig. 4 is a flow chart illustrating a method of dispensing a pharmaceutical according to an embodiment of the present invention.

### DETAILED DESCRIPTION

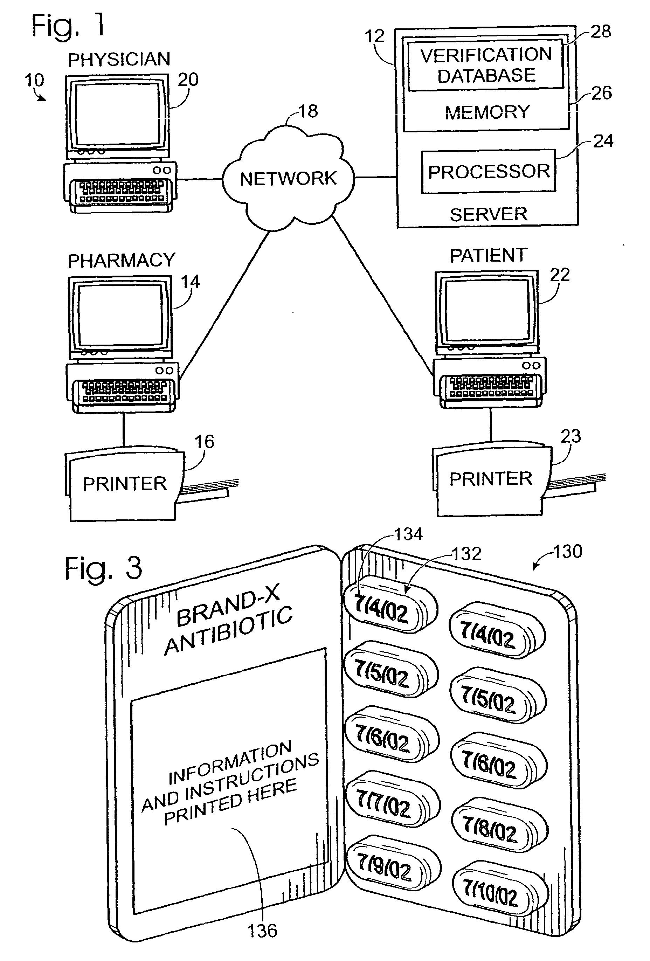

Referring initially to Fig. 1, a system configured to produce a patient-specific pharmaceutical container in accordance with an embodiment of the present invention is indicated generally at 10. System 10, it will be appreciated, may include a server 12, a pharmacy terminal 14, a pharmacy printer 16, and a network communications link 18. System 10 also may include a physician terminal 20 and/or a patient terminal 22, either or both of which may be used to enter patient information and to communicate the information to server 12, or any of the other terminals. A patient printer 23 and/or a physician printer (not shown) also may be provided, either or both of which may be configured to print custom pharmaceutical labels.

Focusing now on server 12, it will be noted that the depicted server includes a processor 24 and memory 26. Processor 24 may be configured to accommodate identification of a pharmaceutical transaction, and to access memory 26 so as to retrieve patient-specific information as will be described further below. The server may be any suitable network server configured to store data, and to be accessed from a proximate or remote terminal or terminals. A single computer, or a set of networked computers, may function as the server. Access to server 12 may be through a user interface on the server itself, or may be through one or more of the designated terminals.

As indicated, a database 28 may be stored in memory 24 of server 12. Alternatively, or additionally, the database may be stored in memory of one or more of the designated terminals, or in memory of separate servers, at proximate or disparate locations. Furthermore, there may be multiple databases stored on any server, or any of the designated terminals. For example, each of a plurality of physicians may maintain a physician-specific database on a single server or designated terminal.

Pharmacy terminal 14 typically is located at a pharmacy in order to aid the pharmacist in record-keeping, communicating with server 12 and/or the other terminals, and producing custom pharmaceutical labels. A typical pharmacy terminal may be a computer terminal including a processor, memory, a user interface, etc. In accordance with the present embodiment of the invention, the pharmacy terminal permits a pharmacist to access server 12, and to retrieve patient information stored on the server for use in production of a custom pharmaceutical label. Other terminals or servers also may be accessed from pharmacy terminal 14 to access patient, physician and/or pharmaceutical information.

A pharmacy printer 16 may be connected to pharmacy terminal 14 for printing custom pharmaceutical labels. Typically, pharmacy printer 16 is configured to handle self-adhesive label blanks, but various forms of media may be employed. Pharmacy printer 16 may be a laser printer, an ink-jet printer, a dot matrix printer, etc., and may be capable of printing in color, or of printing only monochromatic images. Pharmacy printer 16 may be directly connected to pharmacy terminal 14, as shown, but also may be linked through a local area network or similar remote communications link. Pharmacy printer 16 also may be configured to handle different sizes of media, to print labels directly on a container or packaging, and/or to automatically print a custom pharmaceutical label for each pharmaceutical transaction a pharmacist makes.

As indicated above, pharmacy printer 16 may be configured to print directly on a pharmaceutical container. Such as, for example, a blister pack container having discrete chambers, each of which includes a discrete dose of a pharmaceutical. Printing directly on a pharmaceutical container permits discrete labeling of discrete doses of a pharmaceutical in accordance with a treatment regimen of a specific patient, as will be explained below. Physician printers and/or patient printers, such as that shown at 23, may be similarly configured.

As also shown in Fig. 1, physician terminal 20 may communicate patient information to server 12 for storage in database 28 of memory 26. Patient information communicated may include patient-specific identification information (such as name, address, age, sex, height, weight, a patient-identifying image, etc.) and patient-specific prescription information (such as pharmaceuticals prescribed, dosing directives, known allergies, etc.). Typically, the physician terminal may be used to store detailed patient information, including that listed above, among other information relevant to the physician's medical practice. The physician may keep records of every patient treated.

In some instances, only selected portions of the patient information from the physician's records may be communicated to server 12. The information communicated may define a prescription record of each prescription the physician issues. Prescription records, it will be appreciated, may be stored on server 12 in database 28, each such prescription record being associated with both patient-identifying information and prescription-identifying information. The database also may include information about packaging of the prescribed pharmaceutical so as to provide for discrete labeling of discrete doses of the prescribed pharmaceutical as will be described further below.

Patient terminal 22 also may communicate data to server 12, and may request a prescription record from database 28 for use in creating a patient-specific pharmaceutical label for a pharmaceutical. The patient terminal thus may be configured to support a patient printer 23 configured to print patient-specific labels for a patient's pharmaceuticals. Typically, patient printer 23 may be configured to operate in the same manner as described above with reference to pharmacy printer 16. Like pharmacy printer 16, patient printer 23 may be of a type, capable of printing on blister pack containers, or other-style pharmaceutical containers having discrete doses.

It will be understood that while terminals 14, 20, and 22 are illustrated and described as computer terminals each may also include a plurality of networked computers. For example, the physician terminal may include multiple computer terminals connected to a local computer network.

Network communications link 18 typically connects server 12, pharmacy terminal 14, physician terminal 20, and patient terminal 22. The network communications link may be any type of network capable of communicating information. For example, network communications link 18 may be a local area network (LAN), a wide area network (WAN), or any combination of these. Network communications link 18 may use any suitable network architecture enabling terminals 14, 20, and 22 to exchange data with server 12.

In one embodiment, pharmacy terminal 14 may be refused access to server 12 without proper authorization. Authorization may require a pre-approved user name and/or password, or may require submission of the pharmacist's licensing data. Similarly, physician terminal 20 and patient terminal 22 may require an identifying user name and/or password to access server 12, or database 28.

Database 28 may include information related to all Food and Drug Administration (FDA) approved pharmaceuticals and/or medications. Among the information included about each FDA approved pharmaceutical may be important information such as drug-to-drug interactions, common side effects, black-box warnings, etc. Packaging or container configurations of the approved pharmaceuticals also may be included in database 28. The container configurations may be used to configure a printer, such as pharmacy printer 16, patient printer 23, or a similar physician printer (not shown) to print a patient-specific pharmaceutical label suitable for the particular packaging or container configuration. For example, a printer may be configured to print instructional indicia on the pharmaceutical label such that the indicia is accurately positioned with respect to the discrete doses to which such indicia relates.

Information in database 28 may need to be kept current as new pharmaceuticals become available and new packaging configurations become available. A proprietor of system 10 thus may handle periodic updating of the database with information about new pharmaceuticals, packaging configurations, etc. For example, when the FDA approves a new pharmaceutical, the relevant pharmaceutical information (including information on packaging of that pharmaceutical) may be entered into database 28. Typically, weekly or monthly updates to the database will ensure that the information is current. However, such periodic updating may take place more often or less often.

As indicated above, database 28 may include records containing, among other information, a patient-specific therapy schedule for the pharmaceutical. For example, a prescription record may include the identity of the patient receiving a prescription, the dosage and quantity of the prescribed pharmaceutical, a time interval between doses of the prescribed pharmaceutical, a unique identifier for the prescription record, the identity of the doctor or medical service provider that issued the prescription, etc. A pharmacy terminal (or patient terminal) may access database 28 to retrieve prescription records for use in configuring a printer to create a patient-specific pharmaceutical label.

The database may be stored in a specialized server, or may be stored in memory on physician terminal 20. In the present illustration, a prescription that a patient gives to a pharmacist may include a code, or unique identifier, that enables the pharmacist to remotely access database 28 via a communications link such as network communications link 18. This enables a physician to maintain a database of prescription records having limited access.

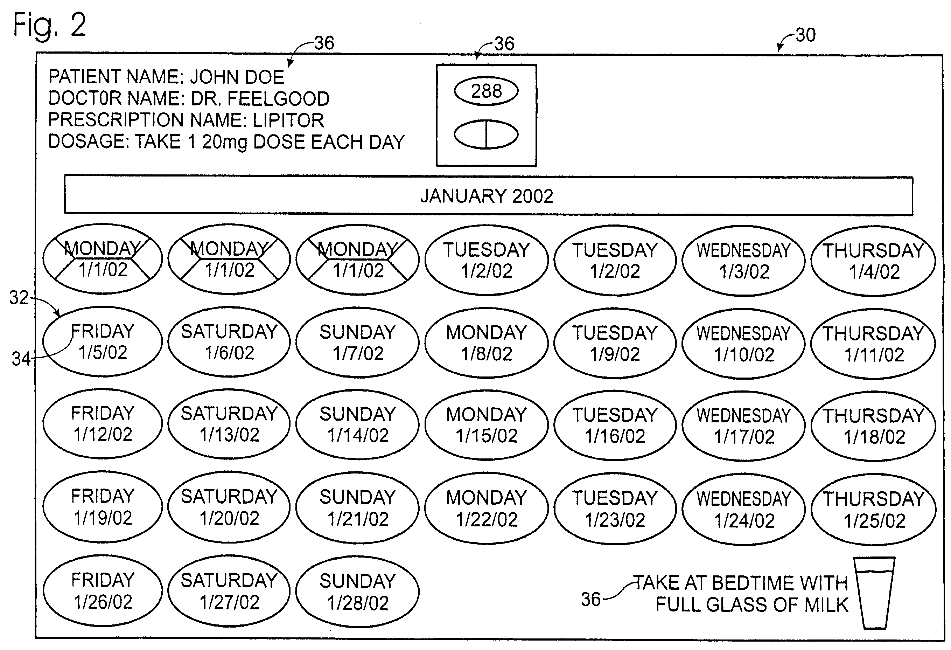

A container 30 according to one embodiment of the present invention is shown in Fig. 2. Container 30 may be configured to include one or more discrete chambers 32 for storing discrete doses of a pharmaceutical. As indicated, instructional indicia 34 may be configured to mark discrete chambers 32 on container 30. The indicia may be printed directly on container 30, or may be printed on a label applied to container 30. In either event, indicia 34 typically is configured to align with the discrete chambers of the container, indicating a time when the contents of the discrete chamber are to be taken by a patient. The time indicated by indicia 34 may be a specific date, such as, "1/1/02", or may be a generic day, such as "Day 1,". Additionally, the indicia may indicate a specific time of day, such as, AM, PM, morning, evening, breakfast, lunch, dinner, etc. For example, as shown in Fig. 2, three doses of a pharmaceutical were administered on the first day of treatments, as indicated by disrupted instructional indicia which labels three doses "Monday 1/1/02". Two doses are to be administered on the second day of treatments as indicated by instructional indicia which labels two doses "Tuesday 1/2/02". The disrupted instructional indicia indicates that the initial dosing has occurred. The unaffected instructional indicia indicates that such dosing is yet to occur in accordance with the indicated instructions.

Some pharmaceutical therapies have special dosing requirements. For example, some Alzheimer medications need to be titrated, that is the dose needs to start out small and be increased to a treatment level to reduce the adverse side effects. By printing approximate indicia in connection with various discrete doses, it is possible to tailor pharmaceutical therapies to the individual patient needs, and to provide for reliable tracking of the desired dosing regimen by the patient.

As shown in Fig. 2, the custom label on container 30 also may include data fields 36 for text, graphic, or other instructional information. One data field, for example, may contain a patient name, a doctor name, a pharmaceutical name, etc. Another data field may include an image of the pharmaceutical, or an image of a patient taking the pharmaceutical. Yet another data field may include dosing instructions, warnings about side effects, and/or other important information about how to administer the pharmaceutical. For example, the data field may include an image of another medication that the patient should not take with the present pharmaceutical and/or an image of food if the pharmaceutical should be taken with food.

With the foregoing in mind, it will be appreciated that a patient-specific pharmaceutical label may be produced for each pharmaceutical transaction using pharmacy terminal 14 and pharmacy printer 16. A pharmaceutical transaction, it will be appreciated, typically occurs when a pharmacist fills or refills a patient's prescription and dispenses the prescribed pharmaceutical to the patient. Similarly, patient terminal 22 and patient printer 23 may produce a patient-specific pharmaceutical label container for a patient using a pharmacy that delivers pharmaceuticals without such a label. This may help such a patient in properly using the pharmaceutical.

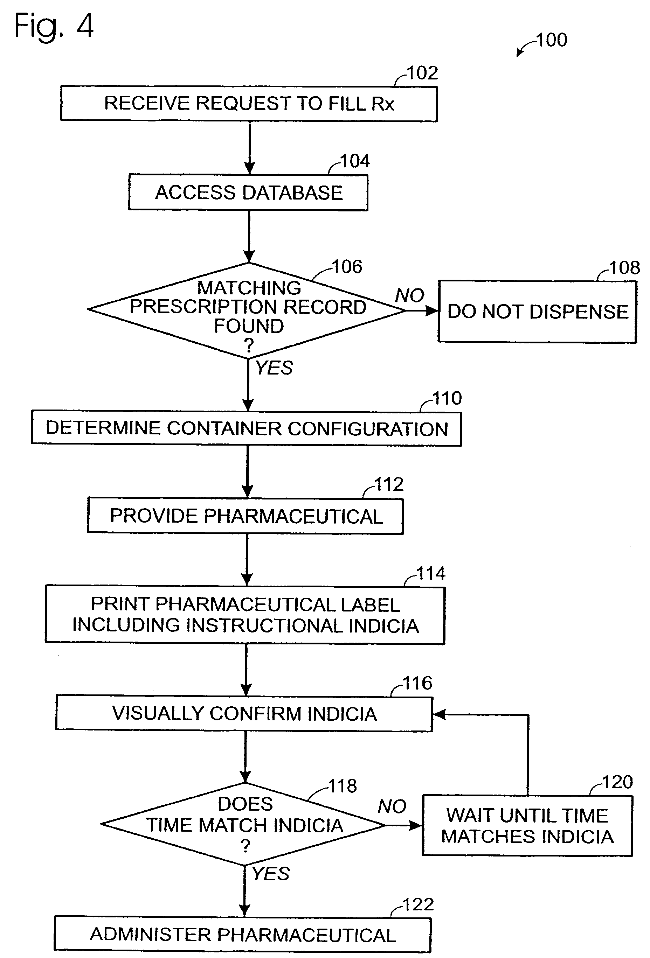

Fig. 3 shows an alternative pharmaceutical container 130, having discrete chambers 132 for storing discrete doses of a pharmaceutical. A patient-specific pharmaceutical label may be applied to and/or printed on container 130 in accordance with one embodiment of the present invention. The patient-specific label may include instructional indicia 134 configured to indicate a time when each discrete dose of the pharmaceutical is to be administered.

As indicated previously, indicia 134 may include a generic time, such as "Day 1", or may indicate a more specific time such as "7/4/02". When a prescription is filled, the patient-specific label may be printed directly on container 130, or printed on a label to be affixed to container 130. Container 130 also may include a data field 136 for printing additional information, such as dosing instructions, warnings, etc. Data field 136 also may include images or graphics as described with respect to Fig. 2 above.

Visual confirmation that the correct dose of a pharmaceutical has been administered may be achieved by examining the discrete chambers and associated indicia of container 130 to ensure that each chamber with indicia indicating a past time is empty, and therefore, that each dose that should have been administered has been administered.

Fig. 4, is a flow chart which schematically illustrates, at 100, a method of using system 10 to verify that prescriptions are accurately administered. Method 100 thus includes receiving a prescription request to fill a prescription (Rx), as shown at 102. The prescription request received by the pharmacist typically specifies both a patient and a pharmaceutical. The prescription request also may include information about the patient receiving the pharmaceutical, the prescribed dosage of the pharmaceutical, the physician who wrote the prescription, and the number of refills. The prescription request also may include a password or code that permits the pharmacist (or a patient) to securely access a prescription record in a database.

The pharmacist (or patient) may access the database, as shown at 104, to verify validity of the prescription request. Verifying a prescription request may include searching the database to determine if a prescription record matching the prescription request exists, as shown at 106. A match may be considered to occur when a unique identifier code from the prescription request is found in a corresponding field of a prescription record contained in the database. Alternatively, a match may be considered to occur if a predetermined number of electronic record fields in an electronic prescription record match corresponding fields of an electronic prescription request (e.g., created by the pharmacist upon receiving a written prescription from a patient). For example, a match may be found if the name of the patient, name of the pharmaceutical, and the prescribed dose are the same in the corresponding fields of the prescription request and the prescription record.

If no match is found, a message may be sent to the pharmacist instructing the pharmacist that there is no matching record in the database, and that the pharmacist should not dispense the pharmaceutical as indicated at 108. The pharmacist may then take further steps to verify the prescription request is valid, such as calling the physician.

If a matching prescription record is found in the database the matching prescription record may be retrieved from the database, and the container configuration may be determined, as shown at 110. The information stored in the prescription record may be used to verify the identity of the patient receiving the pharmaceutical to verify the pharmaceutical and dosing regimen, and to identify the container configuration (e.g., the configuration of discrete chambers of the container). It will be appreciated, for example, that the pharmacist may provide the prescribed pharmaceutical in a container having discrete chambers with discrete doses of the pharmaceutical contained therein, as shown at 112. A container having discrete doses in discrete chambers may prevent contamination, and may provide a convenient way of tracking doses taken as described above. A patient, or third party, thus may track doses taken by identifying empty discrete chambers in the container and/or by reviewing full discrete chambers.

A pharmacist, or a patient, may print a custom pharmaceutical label including instructional indicia associated with each discrete dose (and/or each discrete chamber) to aid in tracking use of a pharmaceutical, as shown at 114. A custom pharmaceutical label may be printed directly on a blister-pack-type container using a printer, such as that shown in Fig. 2, or may be printed on separate print media which may later be affixed to the container. Once the label has been affixed to the container, the pharmaceutical in the container may be administered in accordance with the instructional indicia.

As indicated previously, the custom pharmaceutical label may include information stored in the retrieved prescription record, such as the prescribing physician's name, the prescribing physician's business address, the patient's name and address, the pharmacy's name and address, the date the prescription was last filled, refill status, brief instructions for administering the pharmaceutical, indicia marking discrete chambers indicating the time when the dose of the pharmaceutical should be administered, the name of the pharmaceutical, the manufacturer of the pharmaceutical, and the prescribed dose of the pharmaceutical. Of course, any subset, or combination of the above mentioned information may be included on the custom pharmaceutical label. Additionally, other information not included in the above list may be included on the label.

Where the pharmaceutical label is printed on separate print media, the media may include a peel-away release liner and adhesive layer for affixing the label to a pharmaceutical container. Alternatively, or additionally, tape or some other adhesive material may be employed to affix the label to the pharmaceutical container. A pharmacist thus may affix the printed pharmaceutical label on the pharmaceutical container, as noted above.

A patient receiving the prescription also may track their use of the pharmaceutical by visually checking the indicia aligned with the discrete doses, as shown at 116. The empty chambers indicate the doses that have been taken. Any chambers not empty after the time indicated by the instructional indicia associated with that chamber show that a dose has been missed. A patient thus may check to see if it is time to take a dose of the pharmaceutical, as indicated at 118. If it is not time to take a dose of the pharmaceutical, the patient waits to take the dose until the present time is the time indicated on the indicia, as indicated at 120. If it is time to take another dose, the patient may remove the pharmaceutical from the chamber and administer the pharmaceutical, as indicated at 122.

It should be understood that while the preceding method description focuses on implementation of the present invention in the context of filling prescriptions, this kind of verification and dose tracking system could be employed in connection with both prescription and non-prescription pharmaceuticals. Furthermore, the system may be useful for taking vitamins or other dietary supplements, and such medicaments are to be considered pharmaceuticals for purposes of this disclosure.

Furthermore, while the present embodiment of the invention has been particularly shown and described with particularity, those skilled in the art will understand that many variations may be made therein without departing from the spirit and scope of invention defined in the following claims. The description thus should be understood to include all novel and non-obvious combinations of elements described herein, and claims may be presented in this or a later application to any novel and non-obvious combination of these elements. The foregoing embodiments are illustrative, and no single feature or element is essential to all possible combinations that may be claimed in this, or a later application. Where the claims recite "a" or "a first" element or the equivalent thereof, such claims should be understood to include incorporation of one or more such elements, neither requiring nor excluding two or more such elements.

## Claims

1. A pharmaceutical dispenser system comprising: a container (30) configured to store discrete doses of pharmaceuticals; and instructional indicia (34) configured to align with each discrete dose of pharmaceutical indicating a time such discrete dose is to be administrated in accordance with a patient-specific therapy regimen.

2. The pharmaceutical dispenser system of claim 1, wherein the instructional indicia (34) includes a date on which each discrete dose is to be administered.

3. A method of providing a pharmaceutical label, comprising: receiving a request (102) to dispense a pharmaceutical deliverable in a container having discrete doses of the pharmaceutical; determining a configuration of the discrete doses; and printing instructional indicia (114) configured to align with the determined configuration of discrete doses, such indicia indicating times to administer respective discrete doses of the pharmaceutical.

4. The method of claim 3, wherein the discrete doses are carried in discrete chambers, the instructional indicia being aligned with the discrete chambers of the container to aid a patient in tracking use of the pharmaceutical upon examining which discrete chambers have been emptied.

5. The method of claim 3, wherein the instructional indicia include dates on which the discrete doses are to be administered.

6. A pharmaceutical label produced by a label-making method, comprising: receiving a request (102) to dispense a pharmaceutical deliverable in a container having discrete chambers for discrete doses of the pharmaceutical; determining a configuration of the discrete chambers (110); and printing a pharmaceutical label (114) including instructional indicia configured to align with the determined configuration of discrete chambers, such indicia indicating times to administer respective discrete doses of the pharmaceutical associated with each discrete chamber.

7. A pharmaceutical label produced by the label-making method of claim 6, wherein the instructional indicia aligned with the discrete chambers of the container are configured to aid a patient in tracking use of the pharmaceutical upon examining which discrete chambers remain full.

8. A pharmaceutical dispenser system, comprising: memory (26) configured to store container configuration data, such container configuration data defining positions of discrete doses of pharmaceutical; a processor (24) configured to access the memory (26) to retrieve the container configuration data for use in directing placement of instructional indicia on a pharmaceutical container; and a printer (16, 23) configured to print such instructional indicia on a label for placement on the pharmaceutical container in accordance with the container configuration data to provide discrete instructions relating to discrete doses of the pharmaceutical.

9. The system of claim 8 wherein the memory is further configured to store a pharmaceutical therapy regimen for use in determining placement of instructional indicia directing administration of the pharmaceutical.

10. A method for verifying that a pharmaceutical therapy regimen is accurately followed, comprising: receiving a request to dispense a pharmaceutical deliverable in a container having discrete doses of the pharmaceutical; retrieving the container configuration data for the requested pharmaceutical; printing a pharmaceutical label based on the retrieved container configuration data, such label including instructional indicia configured for alignment with each discrete dose of the pharmaceutical, such instructional indicia indicating a time to administer such discrete doses of the pharmaceutical in accordance with a predetermined pharmaceutical therapy regimen; and visually comparing the time indicated by instructional indicia aligned with a discrete dose with a current time to ensure that such discrete does has been administered as indicated by the instructional indicia.
